# EUROPEAN PATENT APPLICATION

(11) **EP 4 091 617 A1**
(43) Date of publication of application: **23.11.2022**
(21) Application number: 21741345.9
(22) Date of filing: 14.01.2021
(51) Int. Cl.: A61K 35/28, A61K 35/30, A61K 35/34, A61K 35/35, A61K 35/36, A61K 35/50, A61K 35/51, A61K 35/545, A61K 38/17, A61P 43/00, C12N 5/0775

(54) **ANTIFIBROTIC AGENT, AND METHOD FOR PRODUCING EXTRACELLULAR VESICLES HAVING ANTIFIBROTIC ACTION**

(30) Priority: 15.01.2020 JP 2020004765
(71) Applicant: FUJIFILM Corporation, Tokyo 106-8620 (JP)
(72) Inventor: YAMANE Masayuki, Amagasaki-shi, Hyogo 661-0963 (JP); ISHIDOME Takamasa, Amagasaki-shi, Hyogo 661-0963 (JP)
(74) Representative: Klunker IP Patentanwälte PartG mbB
(86) International application number: PCT/JP2021/001131
(87) International publication number: WO 2021/145395

(57) **Abstract**

An object of the present invention is to provide a therapeutic preparation composed of a population of extracellular vesicles having a high therapeutic effect, particularly to provide an anti-fibrosis agent. The present invention relates to the anti-fibrosis agent containing a mesenchymal stem cell-derived extracellular vesicle, as an active ingredient, in which an extracellular vesicle is obtained by a method of using a substance that contains an extracellular vesicle having an affinity for phosphatidylserine, or/and an extracellular vesicle is derived from a mesenchymal stem cell stimulated with fibroblast growth factor, and relates to a method of producing an extracellular vesicle as the anti-fibrosis agent.

## Description

### BACKGROUND OF THE INVENTION

### 1. Field of the Invention

The present invention relates to an anti-fibrosis agent composed of mesenchymal stem cell-derived extracellular vesicles.

### 2. Description of the Related Art

It is considered that extracellular vesicles that are derived from cells and are small membrane vesicles composed of lipid bilayers are responsible for intercellular communication through the transfer of nucleic acids such as mRNA and microRNA contained, or proteins (Mathieu M, Martin-Jaular L, Lavieu G, Thery C (2019) Specificities of secretion and uptake of exosomes and other extracellular vesicles for cell-to-cell communication. Nat Cell Biol, 21(1): 9-17).

Mesenchymal stem cells (hereinafter may be abbreviated as "MSCs") are somatic stem cells derived from mesoderm tissue. MSCs can be separated from adipose, bone marrow, umbilical cord matrix, or the like, all of which are believed to have in common the ability to adhesiveness, CD105, CD73, CD90 positive, CD45, CD34, CD14, CD11b, CD79a, CD19, HLA-Class II (DR)negative, differentiation potential into bone, adipose, and cartilage.

In recent years, it has been suggested that extracellular vesicles may be involved in various diseases. In addition, it has been reported that extracellular vesicles acquired from mesenchymal stem cell-derived extracellular vesicles by an ultracentrifugal method have an anti-fibrosis action (anti-fibrosis effect) (Ferguson et al.(2018)The microRNA regulatory landscape of MSC-derived exosomes: a systems view. Sci Rep,8(1):1419).

### SUMMARY OF THE INVENTION

The present inventors have examined the anti-fibrosis action of extracellular vesicles acquired from mesenchymal stem cell-derived extracellular vesicles by the ultracentrifugal method, and have found that the action was weak and insufficient for industrial use. In view of the above circumstance, an object of the present invention is to provide a therapeutic preparation composed of a population of extracellular vesicles having a higher therapeutic effect, particularly to provide an anti-fibrosis agent.

In order to solve the above problems, the present inventors have diligently studied extracellular vesicles selectively recovered by various methods of acquiring extracellular vesicles. As a result, the present inventors have found that a population of extracellular vesicles having a feature such as PS positivity, which is acquired by a method of using a substance having an affinity for phosphatidylserine (PS) (PS-affinity method) or/and a population of mesenchymal stem cell-derived extracellular vesicles stimulated with fibroblast growth factor is highly effective as an anti-fibrosis agent, and have completed the present invention.

That is, a basic aspect of the present invention includes:
(1) an anti-fibrosis agent comprising a mesenchymal stem cell-derived extracellular vesicle, as an active ingredient, in which an extracellular vesicle is obtained by a method of using a substance having an affinity for phosphatidylserine, or/and an extracellular vesicle is derived from a mesenchymal stem cell stimulated with fibroblast growth factor;
(2) the anti-fibrosis agent according to (1), in which the mesenchymal stem cell is a cell derived from an iPS cell or a cell derived from one or more tissues selected from the group consisting of umbilical cord, umbilical cord blood, bone marrow, adipose, muscle, nerve, skin, amniotic membrane, and placenta;
(3) the anti-fibrosis agent according to (1) or (2), in which the mesenchymal stem cell is stimulated with transforming growth factor β;
(4) the anti-fibrosis agent according to (3), in which the transforming growth factor β is TGFβ1 or TGFβ3;
(5) the anti-fibrosis agent according to any one of (1) to (4), in which the extracellular vesicle is obtained by a method of using a substance having an affinity for phosphatidylserine;
(6) the anti-fibrosis agent according to (5), in which the substance having an affinity for phosphatidylserine is a Tim protein;
(7) the anti-fibrosis agent according to (6), in which the Tim protein is selected from Tim4 protein, Tim3 protein, and Tim1 protein;
(8) the anti-fibrosis agent according to (1) or (2), in which the extracellular vesicle is derived from a mesenchymal stem cell stimulated with transforming growth factor β, and is obtained by a method of using a substance having an affinity for phosphatidylserine.
   The present invention is also a method of producing an extracellular vesicle as the anti-fibrosis agent, and specifically includes:
(9) a method of producing an extracellular vesicle having an anti-fibrosis action, the method comprising a step of obtaining an extracellular vesicle from a mesenchymal stem cell-derived extracellular vesicle by a method of using a substance having an affinity for phosphatidylserine, or/and a step of obtaining a mesenchymal stem cell-derived extracellular vesicle stimulated with transforming growth factor β;
(10) a method of producing an extracellular vesicle according to (9), the method comprising a step of obtaining an extracellular vesicle from a mesenchymal stem cell-derived extracellular vesicle by a method of using a substance having an affinity for phosphatidylserine;
(11) the method of producing an extracellular vesicle according to (10), in which the substance having an affinity for phosphatidylserine is a Tim protein;
(12) the method of producing an extracellular vesicle according to (11), in which the Tim protein is selected from Tim4 protein, Tim3 protein, and Tim1 protein;
(13) The method of producing an extracellular vesicle according to (9) to (12), the method further comprising obtaining a mesenchymal stem cell-derived extracellular vesicle stimulated with transforming growth factor β.

The present invention is to provide the anti-fibrosis agent containing an extracellular vesicle that has an anti-fibrosis action and that is obtained from a mesenchymal stem cell, as an active ingredient. Since the anti-fibrosis agent provided in the present invention has an action of regulating the production of factors involved in tissue fibrosis, for example, collagen III, collagen V, and α-SMA collagen, which are fibrosis-related genes, the progress of fibrosis in living tissues or organs is suppressed. Therefore, it is possible to regenerate functions of the living tissues and the organs that have fallen into functional disorder or that have become dysfunctional.

### BRIEF DESCRIPTION OF THE DRAWINGS

Fig. 1 is a diagram illustrating a result of analysis of a particle size distribution of MSC-derived extracellular vesicles acquired by a PS-affinity method and an ultracentrifugation method by a nanoparticle tracking analysis (NTA) method.
Fig. 2 is a diagram illustrating results of detecting CD9, CD63, and CD81 of MSC-derived extracellular vesicles acquired by the PS-affinity method and the ultracentrifugation method by a Western blotting method.
Fig. 3 is a diagram illustrating primers used for quantitative PCR.
Fig. 4 is a diagram illustrating a result of evaluating an anti-fibrosis action of bone marrow MSC-derived extracellular vesicles using a mRNA expression level of collagen III as an index.
Fig. 5 is a diagram illustrating a result of evaluating an anti-fibrosis action of bone marrow MSC-derived extracellular vesicles using a mRNA expression level of collagen V as an index.
Fig. 6 is a diagram illustrating a result of evaluating an anti-fibrosis action of bone marrow MSC-derived extracellular vesicles using a mRNA expression level of αSMA as an index.
Fig. 7 is a diagram illustrating a result of evaluating an anti-fibrosis action of bone marrow MSC-derived extracellular vesicles using a mRNA expression level of collagen III as an index.
Fig. 8 is a diagram illustrating a result of evaluating an anti-fibrosis action of bone marrow MSC-derived extracellular vesicles using a mRNA expression level of collagen V as an index.
Fig. 9 is a diagram illustrating a result of evaluating an anti-fibrosis action of bone marrow MSC-derived extracellular vesicles using a mRNA expression level of α-SMA as an index.
Fig. 10 is a diagram illustrating a result of evaluating an anti-fibrosis action of each of adipose MSC-derived extracellular vesicles and umbilical cord MSC-derived extracellular vesicles using a mRNA expression level of collagen III as an index.
Fig. 11 is a diagram illustrating a result of evaluating an anti-fibrosis action of each of adipose MSC-derived extracellular vesicles and umbilical cord MSC-derived extracellular vesicles using a mRNA expression level of collagen V as an index.
Fig. 12 is a diagram illustrating a result of evaluating an anti-fibrosis action of each of adipose MSC-derived extracellular vesicles and umbilical cord MSC-derived extracellular vesicles using a mRNA expression level of αSMA as an index.

### DESCRIPTION OF THE PREFERRED EMBODIMENTS

As described above, a basic aspect of the present invention includes an anti-fibrosis agent containing an extracellular vesicle that is obtained by a method of using a substance having an affinity for phosphatidylserine obtained from a mesenchymal stem cell-derived extracellular vesicle or/and a mesenchymal stem cell-derived extracellular vesicle stimulated with transforming growth factor β, as an active ingredient

An extracellular vesicle (hereinafter, may be abbreviated as "EV") is a small membrane vesicle derived from a cell and composed of a lipid bilayer. The extracellular vesicle usually has a diameter of 20 nm to 1000 nm, preferably 50 nm to 800 nm, more preferably 50 nm to 500 nm, and particularly preferably 50 nm to 200 nm. Examples of the extracellular vesicle include extracellular vesicles included in various classifications according to the origin of their occurrence, the size of small membrane vesicles, and the like as described in Nature Reviews Immunology 9,581-593 (August 2009), "Obesity Research" Vol. 13, No. 2, 2007, Topics Naoto Aoki and others. Specific examples thereof include exosomes, microvesicles, ectosomes, membrane particles, exosome-like vesicles, apoptotic vesicles, adiposomes, and the like, exosomes and microvesicles are preferable, and exosomes are more preferable.

The exosomes are cell-derived small membrane vesicles composed of lipid bilayers, and for example, each exosome has a diameter of 50 nm to 200 nm, preferably has a diameter of 50 nm to 150 nm, and more preferably has a diameter of 50 nm to 100 nm. The exosomes are believed to be derived from late endosomes.

The microvesicles are cell-derived small membrane vesicles composed of lipid bilayers, and for example, each microvesicle has a diameter of 100 nm to 1000 nm, preferably has a diameter of 100 nm to 800 nm, and more preferably has a diameter of 100 nm to 500 nm. The microvesicles are believed to be derived from cell membranes.

The MSCs are stem cells having differentiation potential into cells belonging to (mesenchymal) tissues derived from mesoderm such as osteoblasts, adipocytes, muscle cells, and chondrocytes. The MSCs can be separated from adipose, bone marrow, umbilical cord matrix, or the like, and the MSCs used in the present invention (hereinafter, may be abbreviated as "the MSCs according to the present invention") can be obtained by, for example, a method of separating MSCs from tissues derived from mesoderm or a method of inducing MSCs from stem cells such as iPS cells and ES cells. As the MSCs according to the present invention, cells derived from one or more tissues selected from the group consisting of umbilical cord, umbilical cord blood, bone marrow, adipose, muscle, nerve, skin, amniotic membrane, and placenta or cells derived from iPS cells are preferably used. Pretreatments such as recovery, concentration, purification, isolation, dilution with a buffer solution, and filtration sterilization may be performed on the MSCs according to the present invention. These pretreatments may be appropriately performed according to conventional methods. The MSCs according to the present invention are preferably stimulated with transforming growth factor β, and more preferably stimulated with TGFβ1 or TGFβ3.

The extracellular vesicles (hereinafter, may be abbreviated as the "extracellular vesicles according to the present invention") in the anti-fibrosis agent, which are provided in the present invention as an active ingredient, are extracellular vesicles (hereinafter, may be abbreviated as the "PS-positive extracellular vesicles") acquired by isolation from MSC-derived extracellular vesicles according to the present invention by using a substance having an affinity for phosphatidylserine, or/and extracellular vesicles (hereinafter, may be abbreviated as the "stimulated MSC-derived extracellular vesicles") derived from MSCs according to the present invention, stimulated with transforming growth factor β.

The PS-positive extracellular vesicles are PS-positive (PS-containing) extracellular vesicles in which phosphatidylserine on a membrane surface of an extracellular vesicle is considered to be exposed.

As the substance having an affinity for phosphatidylserine (hereinafter, may be abbreviated as a "PS-affinity substance"), any substance may be employed as long as it can be specifically bonded to phosphatidylserine composing a membrane of an extracellular vesicle. Examples thereof include Annexin V; MFG-E8; Tim proteins such as Tim1 (T cell immunoglobulin mucin domain-containing molecule 1, T-cell immunoglobulin-mucin-domain 1) protein, Tim2 (T cell immunoglobulin·mucin domain-containing molecule 2, T-cell immunoglobulin-mucin-domain 2) protein, Tim3 (T cell immunoglobulin mucin domain-containing molecule 3, T-cell immunoglobulin-mucin-domain 3) protein, and Tim4 (T cell immunoglobulin·mucin domain-containing molecule 4, T-cell immunoglobulin-mucin-domain 4) protein, and from the viewpoint that extracellular vesicles can be efficiently acquired, Tim proteins are preferable, it is more preferable to be selected from Tim4 protein, Tim3 protein, and Tim1 protein, Tim4 protein and Tim1 protein are still more preferable, and Tim4 protein is particularly preferable.

The PS-positive extracellular vesicles are obtained by isolation from a cell culture supernatant liquid of the MSCs according to the present invention, which contains EV (hereinafter, may be abbreviated as a "cell culture supernatant liquid containing EV") using a substance having an affinity for phosphatidylserine, or by acquiring EV from a cell culture supernatant liquid of the MSCs according to the present invention, which contains EV, by a conventional method such as an ultracentrifugal method in this field, and isolating the PS-positive extracellular vesicles using a substance having an affinity for phosphatidylserine from the obtained EV. Among these, it is preferable to directly isolate extracellular vesicles from the cell culture supernatant liquid of the MSCs according to the present invention, which contains EV, using a substance having an affinity for phosphatidylserine. The MSCs according to the present invention are proliferated by cell culture, and the proliferated cells are further cultured in an EV production culture medium to obtain the cell culture supernatant liquid containing EV. The MSC cell culture according to the present invention and the culture in the EV production culture medium may be carried out according to a conventional method carried out in this field, and the medium and culture conditions used are not particularly limited.

The outline of a method of acquiring extracellular vesicles by the method in which a substance having an affinity for phosphatidylserine (hereinafter, may be abbreviated as a "PS-affinity method") is used is described below. As the PS-affinity method, for example, a specific example is described in WO2016/088689A.

The PS-affinity method including brining the cell culture supernatant liquid containing EV into contact with a PS-affinity substance in the presence of calcium ions, forming a composite body (hereinafter, may be abbreviated as the "composite body according to the present invention") by combining the extracellular vesicles in the cell culture supernatant liquid with the PS-affinity substance, and separating the PS-affinity substance from the composite body to acquire PS-positive extracellular vesicles.

Specifically, a preferable method as the PS-affinity method includes following steps:
(1) a step of brining the cell culture supernatant liquid containing EV into contact with a PS-affinity substance in the presence of calcium ions, forming a composite body (the composite body according to the present invention) by combining the PS-positive extracellular vesicles in the cell culture supernatant liquid with the PS-affinity substance (hereinafter, may be abbreviated as the "composite body formation step");
(2) a step of separating the composite body according to the present invention obtained in the composite body formation step from the cell culture supernatant liquid containing EV (hereinafter, may be abbreviated as the "composite body separation step");
(3) a step of acquiring PS-positive extracellular vesicles by separation of the PS-positive extracellular vesicles from the composite body according to the present invention (hereinafter, may be abbreviated as the "acquisition step").

The cell culture supernatant liquid containing EV and the PS-affinity substance in the PS-affinity method are the same as those described above, and preferred and specific examples are also the same.

The PS-affinity substance used in the composite body formation step is preferably a substance immobilized (bonded) to an insoluble carrier. In this case, the composite body according to the present invention can be separated from the cell culture supernatant liquid containing EV by a known B/F separation method in the composite body separation step.

An example of a method of immobilizing a PS-affinity substance on an insoluble carrier will be described below, and a composite body can be obtained by, for example, a method described in WO2016/088689A.

Examples of the insoluble carrier for immobilizing the PS-affinity substance include an insoluble carrier used in an immunoassay method. Specific examples thereof include organic substances such as polystyrene, polyacrylic acid, polymethacrylic acid, polymethyl methacrylate, polyacrylamide, polyglycidylmethacrylate, polypropylene, polyolefin, polyimide, polyurethane, polyester, polyvinyl chloride, polyethylene, polychlorocarbonate, a silicone resin, silicone rubber, agarose, dextran, and ethylene-maleic anhydride copolymer; inorganic substances such as glass, silicon oxide, silicon, porous glass, ground glass, alumina, silica gel, and metal oxides; magnetic materials such as iron, cobalt, nickel, magnetite, and chromite; and substances prepared from alloys of these magnetic materials. Examples of the usage form of these carriers include particles (beads), microplates, tubes, disc-shaped pieces, and the like. The form of the insoluble carriers is preferably particles (beads), and the size of each of the particles is not particularly limited, but for example, those have a size of 10 nm to 100 µm, and those preferably have a size of 100 nm to 10 µm.

Examples of a method of bonding the PS-affinity substance to the insoluble carrier include a method known per se for bonding a protein to a carrier. Examples of the method include a bonding method by affinity bonding, a bonding method by chemical bonding (for example, methods described in JP3269554B, WO2012/039395A), a bonding method by physical adsorption (for example, a method described in JP1993-41946B (JP-H05-41946)), or the like, the bonding method by physical adsorption and the bonding method by affinity bonding are preferable, and from the viewpoint of facilitation, the bonding method by physical adsorption is more preferable.

As a method of bonding the PS-affinity substance to the insoluble carrier by physical adsorption, the PS-affinity substance and the insoluble carrier are brought into contact with each other under a condition in which the PS-affinity substance and the insoluble carrier are bound, according to a method known per se.

The amount of the PS-affinity substance to be bonded to the insoluble carrier may be, for example, 0.1 µg to 50 µg, is preferably 0.1 µg to 30 µg, and is more preferably 0.1 µg to 20 µg, with respect to 1 mg of the insoluble carrier in a case where the insoluble carrier is the form of particles (beads), for example.

The physical adsorption of the PS-affinity substance and the insoluble carrier may be carried out, for example, by bringing a solution containing the PS-affinity substance into contact with the insoluble carrier.

In the solution containing the PS-affinity substance, a solution for dissolving the PS-affinity substance may be any solution as long as being capable of dissolving the PS-affinity substance in a stable state, and examples thereof include purified water, a buffer solution having a buffering action at pH 6.0 to 9.8, preferably 7.0 to 9.6 (for example, a Good's buffer solution, such as MOPS, or the like, a carbonate buffer solution, PBS, TBS, TBS-T, HBS or the like). The buffer agent concentration in these buffer solutions may be appropriately selected from a range of usually 5 to 100 mM, and preferably 10 to 100 mM. In a case where NaCl is added, the NaCl concentration is, for example, 100 to 200 mM, and is preferably 140 to 160 mM. Saccharides, salts such as NaCl, surfactants such as Tween (trademark) 20, preservatives, proteins, or the like may be contained in the solution containing the PS-affinity substance, as long as an amount thereof does not inhibit the bonding of the PS-affinity substance and the insoluble carrier.

Specific examples of the method of bonding the PS-affinity substance to the insoluble carrier by physical adsorption include the following methods. For example, 1 mg of a bead (particle) carrier and the solution containing the PS-affinity substance of 0.1 µg to 50 µg, preferably 0.1 µg to 30 µg, more preferably 0.1 µg to 20 µg are brought into contact with each other, and reacted with each other at 2°C to 37°C, preferably 4°C to 11°C for 0.5 to 48 hours, preferably 0.5 to 24 hours.

The insoluble carrier to which the PS-affinity substance obtained as described above is immobilized may be subjected to a blocking treatment usually performed in this field.

The composite body formation step is carried out in the presence of calcium ions. Calcium ions are present in a case where the PS-affinity substance is brought into contact with the PS-positive extracellular vesicles in the cell culture supernatant liquid containing EV. The concentration of calcium ions in a case where the PS-affinity substance is brought into contact with the PS-positive extracellular vesicles in the cell culture supernatant liquid containing EV is usually 0.5 mM to 100 mM, preferably 1.0 mM to 10 mM, and more preferably 2.0 mM to 5.0 mM. The above described concentration of calcium ions is required in the solution containing the composite body according to the present invention until the composite body according to the present invention is formed by bringing the PS-affinity substance into contact with the PS-positive extracellular vesicles in the cell culture supernatant liquid containing EV and the composite body separation step is carried out, that is, until the step of separating the composite body according to the present invention is carried out.

What the calcium ions are derived from is not particularly limited, and examples thereof include calcium chloride, calcium hydroxide, calcium hydrogencarbonate, calcium iodide, calcium bromide, and calcium acetate, calcium chloride, calcium hydrogencarbonate, and calcium iodide are preferable, and calcium chloride and calcium hydrogencarbonate are more preferable.

As a method of allowing calcium ions to be present in a case where the PS-affinity substance is brought into contact with the PS-positive extracellular vesicles in the cell culture supernatant liquid containing EV, the calcium ion as described above may be contained in the cell culture supernatant liquid containing EV or/and the solution containing the PS-affinity substance so that the concentration of calcium ions in a case where the PS-affinity substance is brought into contact with the PS-positive extracellular vesicles in the cell culture supernatant liquid containing EV is in the above described range. In addition, a solution containing the amount of calcium ions in which the concentration of calcium ions in a case where the PS-affinity substance is brought into contact with the PS-positive extracellular vesicles in the cell culture supernatant liquid containing EV is in the above described range (hereinafter, may be abbreviated as the "calcium ion-containing solution"), the cell culture supernatant liquid containing EV, and the solution containing the PS-affinity substance may be mixed with each other.

In the calcium ion-containing solution according to the present invention, a solution for dissolving calcium ions may be any solution that does not inhibit bonding of the PS-positive extracellular vesicles and the PS-affinity substance, and examples thereof include water and a buffer solution having a buffering action at pH 7.0 to pH 8.0, and a buffer solution having a buffering action at pH 7.2 to pH 7.6 (for example, TBS, HBS, or the like) is preferable. A phosphate buffer is not preferable since the phosphate buffer and calcium are bonded to cause precipitation. The buffer agent concentration in these buffer solutions is appropriately selected from a range of usually 5 mM to 50 mM, and preferably 10 mM to 30 mM. In a case where NaCl is added, the NaCl concentration is usually selected from the range of 100 mM to 200 mM, and appropriately selected from the range of 140 mM to 160 mM.

For example, saccharides, salts such as NaCl, surfactants, preservatives, proteins such as BSA, or the like may be contained in the calcium ion-containing solution according to the present invention as long as an amount thereof does not inhibit the bonding of the PS-positive extracellular vesicles and the PS-affinity substance. Examples of the surfactants include Tween (trademark) 20 and the like, and the concentration of a surfactant in the calcium ion-containing solution according to the present invention is usually 0.00001% to 0.2%, and preferably usually 0.0005% to 0.1%.

In the composite body formation step, the amount of the cell culture supernatant liquid containing EV to be brought into contact with 1 µg of the PS-affinity substance (that may be immobilized on the insoluble carrier) is usually 0.1 ml to 100 ml, preferably 0.1 ml to 10 ml, and more preferably 0.1 ml to 1.0 ml. The temperature at which the cell culture supernatant liquid containing EV is brought into contact with the PS-affinity substance is usually 2°C to 37°C, preferably 4°C to 37°C, and more preferably 4°C to 30°C. The time when the cell culture supernatant liquid containing EV is being brought into contact with the PS-affinity substance is usually 0.5 to 24 hours, preferably 0.5 to 8 hours, and more preferably 0.5 to 4 hours.

In a case where the insoluble carrier on which the PS-affinity substance is immobilized is used in the composite body formation step, the amount of the carriers is usually 0.1 mg to 20 mg per 1 mL of a solution for forming the composite body according to the present invention, preferably 0.3 mg to 10 mg, and more preferably 0.5 mg to 6.0 mg.

The composite body formation step may be performed by, for example, the following method. That is, the insoluble carriers on which the PS-affinity substance is immobilized in an amount of usually 0.1 mg to 20 mg, preferably 0.3 mg to 10 mg, and more preferably 0.5 mg to 6.0 mg per 1 mL of a solution after the cell culture supernatant liquid containing EV, the insoluble carriers on which the PS-affinity substance is immobilized, and the calcium ion-containing solution according to the present invention are mixed with each other, the calcium ion-containing solution according to the present invention in an amount such that the calcium ion concentration in a solution after the cell culture supernatant liquid containing EV, the carriers, and the calcium ion-containing solution are mixed with each other is usually 0.5 mM to 100 mM, preferably 1.0 mM to 10 mM, and more preferably 2.0 mM to 5.0 mM, and the cell culture supernatant liquid containing EV in an amount of usually 0.1 ml to 100 ml, preferably 0.1 ml to 10 ml, and more preferably 0.1 ml to 1.0 ml per 1 mg of the insoluble carrier on which the PS-affinity substance is immobilized are brought into contact with each other at usually 4.0°C to 37°C, preferably 4.0°C to 25°C, and more preferably 4.0°C to 11°C for usually 0.5 to 24 hours, preferably 0.5 to 8.0 hours, and more preferably 0.5 to 4.0 hours, to form a composite body composed of the PS-affinity substance bonded to the carrier and the PS-positive extracellular vesicle in the cell culture supernatant liquid containing EV (composite body according to the present invention).

The composite body separation step may be any method as long as the composite body according to the present invention and the cell culture supernatant liquid containing EV can be separated from each other to obtain the composite body according to the present invention, and examples thereof include the following methods.
(1) In a case where in an insoluble carrier on which a PS-affinity substance is immobilized, the carrier is a magnetic carrier: a method of installing a container that contains the composite body according to the present invention obtained in the composite body formation step on a magnet stand, as necessary, aggregating the composite body according to the present invention on a tube wall by using magnetic force, and removing supernatant to separate the composite body from the cell culture supernatant liquid containing EV.
(2) In a case where in an insoluble carrier on which a PS-affinity substance is immobilized, the carrier in the form of a bead: a method of centrifuging a container that contains the composite body according to the present invention obtained in the composite body formation step, aggregating the composite body according to the present invention by precipitation, and removing supernatant to separate the composite body from the cell culture supernatant liquid containing EV.
(3) A method of separating the composite body according to the present invention from the cell culture supernatant liquid containing EV by filtration.

Specific examples of the composite body separation step include the following method:
in a case where a magnetic carrier is used as an insoluble carrier, a method of installing a container that has been subjected to the composite body formation step on a magnet stand, as necessary, aggregating the obtained composite body according to the present invention on a tube wall by using magnetic force, and removing a supernatant specimen.

After the composite body separation step, as necessary, the obtained composite body according to the present invention may be washed with a calcium ion-containing washing solution (hereinafter, may be abbreviated as a "washing operation"). By the washing operation, impurities in a biological specimen such as cell-derived components adhering to a surface of the insoluble carrier on which the PS-affinity substance is immobilized can be removed. As a washing method, other than a calcium ion-containing washing solution being used, a washing method usually used in this field can be used.

The calcium ion-containing washing solution used in the washing operation may be any solution as long as the solution contains calcium ions of usually 0.5 to 100 mM, preferably 1 to 10 mM, more preferably 2 mM to 5 mM, and does not affect the bonding of the PS-positive extracellular vesicles and the PS-affinity substance immobilized on an insoluble carrier, and examples thereof include a buffer solution (for example, TBS, TBS-T, and HBS) that contains calcium ions of usually 0.5 mM to 100 mM, preferably usually 1 mM to 10 mM, more preferably usually 2 mM to 5 mM, that has a buffering action at pH 7.0 to pH 8.0, preferably at pH 7.2 to pH 7.6, and that does not precipitate calcium. A phosphate buffer is not preferable since the phosphate buffer and calcium are bonded to cause precipitation. A buffer agent concentration in these buffer solutions is usually appropriately selected from the range of 5 mM to 50 mM, preferably 10 mM to 30 mM, and the concentration in a case of containing NaCl is usually appropriately selected from the range of 100 mM to 200 mM, and preferably 140 mM to 160 mM. Saccharides, salts such as NaCl, surfactants, preservatives, proteins such as BSA, or the like may be contained in this solution as long as an amount thereof does not inhibit the bonding of the PS-positive extracellular vesicles and the PS-affinity substance immobilized on the insoluble carrier. Examples of the surfactants include Tween (trademark) 20 (FUJIFILM Wako Pure Chemical Corporation) and the like, and a concentration of a surfactant in the washing solution is usually 0.00001% to 0.2%, and preferably usually 0.0005% to 0.1%.

A specific example of the washing operation will be described as an example of a washing operation using magnetic particles as an insoluble carrier for immobilizing the PS-affinity substance. That is, the calcium ion-containing washing solution according to the present invention is added to the container containing the composite body according to the present invention obtained in the composite body separation step, and the mixture is stirred. Thereafter, the container is installed on a magnet stand, the composite body according to the present invention is aggregated on the tube wall using magnetic force, and the solution in the container is discarded. These washing operations may be repeated several times as needed.

The acquisition step may be any method as long as the PS-positive extracellular vesicles can be acquired from the composite body according to the present invention, and a method by which the calcium ion concentration is reduced is preferable. Examples of the method by which the calcium ion concentration is reduced include a method of using a calcium ion chelating agent. That is, after the composite body separation step, and as necessary, after the washing operation, the PS-positive extracellular vesicles may be separated from the composite body according to the present invention by the calcium ion chelating agent acting on calcium ions (calcium ions bonded to the composite body according to the present invention and calcium ions introduced from the solution containing the composite body according to the present invention) to chelate calcium ions in a reaction system to reduce an effective concentration of the calcium ions in the reaction system.

The calcium ion chelating agent used in this method may be any compound capable of chelating calcium ions, and examples thereof include ethylenediaminetetraacetic acid (EDTA), nitrilotriacetic acid (NTA), diethylenetriaminepentaacetic acid (DTPA), L-glutamic acid diacetic acid (GLDA), hydroxyethylethylenediaminetriacetic acid (HEDTA), ethylene glycol bis (β-aminoethyl ether)-N,N,N,N-tetraacetic acid (GEDTA), triethylenetetramine-N,N,N',N",N‴,N‴-hexacetic acid (TTHA), 2-hydroxyethyliminodiacetic acid (HIDA), N,N-bis(2-hydroxyethyl)glycine (DHEG), trans-1z,2-diaminocyclohexane-N,N,N',N'- tetraacetic acid, monohydrate (CyDTA), and EDTA, GEDTA, CyDTA are preferable.

The calcium ion chelating agent is usually used as a solution. A solution for dissolving the calcium ion chelating agent may be any solution as long as the solution dissolves the calcium ion chelating agent, and examples thereof include purified water, a buffer solution, and the like. As the buffer solution, a buffer solution having a buffering action usually at pH 7.0 to pH 8.0, and preferably pH 7.2 to pH 7.6 (for example, PBS, TBS, HBS, or the like) is preferable. A buffer agent concentration in these buffer solutions is usually appropriately selected from the range of 5 Mm to 50 Mm, preferably 10 Mm to 30 Mm, and the concentration in a case of containing NaCl is usually appropriately selected from the range of 100 Mm to 200 Mm, and preferably 140 Mm to 160 Mm. The solution containing a calcium ion chelating agent (hereinafter, may be abbreviated as "calcium ion chelating agent-containing solution") may contain, for example, saccharides, salts such as NaCl, preservatives, proteins, and the like.

The concentration of the calcium ion chelating agent in the calcium ion chelating agent-containing solution is usually 0.5 mM to 500 mM, preferably 0.5 mM to 100 mM, and more preferably 0.5 mM to 50 mM. The pH of the calcium ion chelating agent-containing solution is usually pH 6.0 to pH 9.0, preferably pH 7.0 to pH 8.0, and more preferably pH 7.2 to pH 7.6.

In order to cause the calcium ion chelating agent to act on the calcium ions bonded to the composite body according to the present invention, the calcium ion chelating agent-containing solution is brought into contact with the composite body (for example, in the form of a pellet) according to the present invention to react the calcium ions bonded to the composite body according to the present invention with the calcium ion chelating agent in the calcium ion chelating agent-containing solution.

The calcium ion chelating agent-containing solution and the composite body according to the present invention can be brought into contact with each other by, for example, a method of suspending the composite body according to the present invention in the calcium ion chelating agent-containing solution (in the case where in an insoluble carrier on which the PS-affinity substance is immobilized, the insoluble carrier is a bead, or the like), a method of immersing the composite body according to the present invention in the calcium ion chelating agent-containing solution (in the case where in an insoluble carrier on which the PS-affinity substance is immobilized, the insoluble carrier is a disc-shaped piece, a tube, or the like), or the like.

As for the amount of the calcium ion chelating agent-containing solution to be brought into contact with the composite body according to the present invention, the amount by which the concentration of the calcium ions in the solution after being brought into contact with the composite body according to the present invention is less than the effective concentration, and the extracellular vesicles are separated from the composite body according to the present invention, may be adopted.

The temperature and time for allowing the calcium ion chelating agent to act (contact) on the composite body according to the present invention are usually 4.0°C to 37°C, preferably 10°C to 30°C, and more preferably 20°C to 30°C, and usually 1 to 10 minutes, and preferably 5 to 15 minutes, respectively.

The acquisition step will be described with an example of a method using a carrier (Tim carrier) in which a Tim protein is bonded to an insoluble carrier, as follows.
That is, after the composite body separation step is carried out, and as necessary, after the washing operation is further carried out, a solution containing the calcium ion chelating agent of usually 0.5 mM to 500 mM, preferably 0.5 mM to 100 mM, and more preferably 0.5 mM to 50 mM is added to the obtained composite body according to the present invention at usually 10 µL to 500 µL, preferably 20 µL to 200 µL, more preferably 50 µL to 100 µL per 1 mg of a Tim carrier, and the reaction is carried out at usually 4.0°C to 37°C, preferably 10°C to 30°C, and more preferably 20°C to 30°C for usually 1 to 30 minutes, preferably 5 to 15 minutes to separate PS-positive extracellular vesicles from the composite body according to the present invention.

By carrying out the acquisition step, the calcium ion chelating agent-containing solution brought into contact with the composite body according to the present invention contains the insoluble carrier on which the PS-affinity substance is immobilized and the extracellular vesicles separated (liberated) from the composite body according to the present invention. Therefore, in a case where the carrier on which the PS-affinity substance is immobilized is removed from the solution, and only the solution is recovered, a solution containing PS-positive extracellular vesicles can be obtained.

The stimulated MSC-derived extracellular vesicles are obtained by isolation from a cell culture supernatant liquid of the MSCs according to the present invention, which contains EV (that is, the cell culture supernatant liquid containing EV to which stimulation is applied by transforming growth factor β) stimulated with transforming growth factor β.

A method of acquiring the stimulated MSC-derived extracellular vesicles may be any conventional method used in a case of isolating EV from a sample, and examples thereof include an affinity method (for example, PS-affinity method), a fractional centrifugation method (for example, a pellet down method, a sucrose cushioning method, an ultracentrifugal method such as density gradient centrifugation method), an immunoprecipitation method, a chromatography method (for example, an ion exchange chromatography method, a gel permeation chromatography method), a density gradient method (for example, a sucrose density gradient method), an electrophoresis method (for example, an organella electrophoresis method), a magnetic separation method (for example, a magnetically activated cell sorting (MACS) method), an ultrafiltration concentration method (for example, a nanofilm ultrafiltration concentration method), a percol gradient isolation method, a method using a microfluidic device, a PEG precipitation method, and the like, an affinity method with which extracellular membrane vesicles having a high degree of purity can be obtained or a fractional centrifugation method that enables theoretically unbiased recovery is preferable, an affinity method or an ultracentrifugal method is more preferable, and an affinity method is particularly preferable. Among the affinity methods, the PS-affinity method is preferable. The affinity method and the fractional centrifugation method may be carried out, for example, based on the method described in JP2016-088689A. One of these isolation methods may be used alone, or two or more methods may be used in combination. In addition, isolation by one isolation method may be repeated twice or more.

The cell culture supernatant liquid of the MSCs according to the present invention, which contains EV and to which stimulation is applied by transforming growth factor β, is obtained by, for example, proliferating the MSCs according to the present invention stimulated with transforming growth factor β by cell culture and by further culturing the proliferated cells in an EV production culture medium.

The transforming growth factor is not particularly limited, and TGFβ1 and TGFβ3 are preferable. The stimulation of the MSCs according to the present invention with transforming growth factor β may be performed by culturing the MSCs according to the present invention in the coexistence of transforming growth factor β.

The MSC cell culture according to the present invention and the culture in the EV production culture medium may be carried out according to a conventional method carried out in this field, and the medium and culture conditions used are not particularly limited.

The extracellular vesicles according to the present invention are preferably PS-positive extracellular vesicles, more preferably extracellular vesicles that are derived from the MSCs according to the present invention to which stimulation is applied by transforming growth factor β, and that are obtained by a method (PS-affinity method) using a substance having an affinity for phosphatidylserine, still more preferably extracellular vesicles that are derived from the MSCs according to the present invention stimulated with transforming growth factor β and that are obtained by a PS-affinity method in which a Tim protein is used, particularly preferably extracellular vesicles that are derived from the MSCs according to the present invention stimulated with TGFβ1 or TGFβ1 and that are obtained by the PS-affinity method of using Tim4 protein, Tim3 protein, or Tim1 protein, and most preferably extracellular vesicles that are derived from the MSCs according to the present invention stimulated with TGFβ1 or TGFβ1 and that are obtained by the PS-affinity method of using Tim4 protein.

The extracellular vesicles according to the present invention thus prepared had an action of regulating the production of factors involved in tissue fibrosis, for example, collagen III, collagen V, α-SMA collagen, which are fibrosis-related genes. Therefore, the anti-fibrosis agent provided in the present invention is based on an action of regulating the production, for example, collagen III, collagen V, and α-SMA collagen, which are fibrosis-related genes, due to the extracellular vesicles according to the present invention, as an active ingredient, and it is possible to regenerate functions of the living tissues and the organs that have fallen into functional disorder or that have become dysfunctional by suppressing the progress of fibrosis in living tissues or organs.

The anti-fibrosis agent provided in the present invention can be an effective therapeutic medication for diseases that can cause fibrosis, for example, pulmonary fibrosis, idiopathic pulmonary fibrosis, retroperitoneal fibrosis, bone marrow fibrosis, rheumatoid arthritis, liver fibrosis, liver cirrhosis, renal fibrosis (renal fibrosis), tubular interstitial nephritis, chronic pancreatitis, scirrhus stomach cancer, pancreatic cystic fibrosis, myocardial fibrosis, endocardial fibrosis, urinary tract tuberculosis and interstitial cystitis, cystitis such as radiation cystitis, postoperative scar, burn scar, keroid, hypertrophic scar, pachyderma, hysteromyoma, prostatic hypertrophy, Alzheimer's disease, sclerosing peritonitis, type I diabetes, organ adhesion after surgery, and the like.

The anti-fibrosis agent provided in the present invention basically contains the extracellular vesicles according to the present invention obtained by isolation from the MSC-derived extracellular vesicles, as an active ingredient, and the dosage form for administration is a solution containing the extracellular vesicles according to the present invention as is, or a pharmaceutically acceptable carrier as needed, or a form formulated as a liquid agent, a suspending agent, a lipo agent, or the like with an additive, or furthermore, a form formulated as a solid agent such as a tablet with a pharmaceutically acceptable additive, as a powder by freeze-drying.

Examples of such carriers and additives include tonicity adjusting agents, thickeners, saccharides, sugar alcohols, antiseptics (preservatives), bactericides or antibacterial agents, pH adjusters, stabilizers, chelating agents, oleaginous bases, gel bases, surfactants, suspending agents, bonding agents, excipients, lubricants, disintegrants, foaming agents, fluidizing agents, dispersants, emulsifiers, buffers, solubilizing agents, antioxidants, sweeteners, acidulating agents, colorants, flavoring agents, perfumes, and cooling agents, but not limited thereto.

Examples of typical carriers, additives, and the like can include the following. Examples of the carriers include an aqueous carrier such as water and hydrous ethanol. Examples of the tonicity adjusting agents include inorganic salts such as sodium chloride, potassium chloride, calcium chloride, and magnesium chloride. Examples of the polyhydric alcohols include glycerin, propylene glycol, polyethylene glycol, and the like. Examples of the thickeners include carboxyvinyl polymer, hydroxyethyl cellulose, hydroxypropylmethyl cellulose, methyl cellulose, alginic acid, polyvinyl alcohol (completely or partially saponified), polyvinylpyrrolidone, macrogol, and the like.

Examples of the saccharides include cyclodextrin, glucose, fructose, lactose, and the like. Examples of sugar alcohols include sugar alcohols such as xylitol, sorbitol, and mannitol. Examples of antiseptics, bactericides, or antibacterial agents include dibutylhydroxytoluene, benzalkonium chloride, benzethonium chloride, chlorhexidine gluconate, sodium dehydroacetate, methyl paraoxybenzoate, ethyl paraoxybenzoate, propyl paraoxybenzoate, butyl sugar paraoxybenzoate. Examples of the pH adjusters include hydrochloric acid, boric acid, aminoethylsulfonic acid, citric acid, acetic acid, sodium hydroxide, potassium hydroxide, calcium hydroxide, magnesium hydroxide, sodium hydrogencarbonate, sodium carbonate, borosand, triethanolamine, monoethanolamine, diisopropanolamine, sulfuric acid, magnesium sulfate, phosphoric acid, polyphosphate, propionic acid, oxalic acid, gluconic acid, fumaric acid, lactic acid, tartaric acid, malic acid, succinic acid, and the like.

Examples of the stabilizers include dibutylhydroxytoluene, trometamol, sodium formaldehyde sulfoxylate (rongalite), tocopherol, sodium pyrosulfite, monoethanolamine, aluminum monostearate, glycerin monostearate, sodium hydrogen sulfite, sodium sulfite, and the like. Examples of the bases include vegetable oils such as olive oil, corn oil, soybean oil, sesame oil, and cottonseed oil; oleaginous bases such as medium-chain fatty acid triglycerides; aqueous bases such as Macrogol 400; gel bases such as carboxyvinyl polymers and gums. Examples of the surfactants include polysorbate 80, hardened castor oil, glycerin fatty acid ester, sorbitan sesquioleate, and the like, and examples of the suspending agents include white beeswax and various surfactants, gum arabic, gum arabic powder, xanthan gum, soy lecithin, and the like.

Furthermore, examples of the bonding agents include hydroxyethyl cellulose, hydroxypropyl cellulose, hydroxypropylmethyl cellulose, sodium carboxymethyl cellulose, polyvinylpyrrolidone, polyvinyl alcohol, and the like, examples of the excipients include sucrose, lactose, starch, corn starch, crystalline cellulose, light anhydrous silicic acid, and the like, examples of the lubricants include sucrose fatty acid ester, magnesium stearate, talc, and the like, examples of the disintegrants include low-substituted hydroxypropyl cellulose, crospovidone, croscarmellose sodium, and the like, and examples of the fluidizing agents include sodium aluminometasilicate, light anhydrous silicic acid, and the like.

The anti-fibrosis agent provided in the present invention is preferably formulated as a liquid agent, a suspending agent, or a lipo agent, and is basically obtained by mixing the solution containing the extracellular vesicles according to the present invention isolated from MSC-derived extracellular vesicles with, for example, saline, 5% glucose solution, lipo emulsion, or the like together with the above described carriers and additives, as necessary. In addition, freeze-dried powder can be used for dissolution before use or the suspension-type preparation.

In a case where the anti-fibrosis agent provided in the present invention is a liquid agent, a suspending agent, or a lipo agent, the pH of the anti-fibrosis agent is not particularly limited as long as the pH thereof is within a pharmaceutically, pharmacologically or physiologically acceptable range, and examples of the range include a range of pH 2.5 to 9.0, preferably 3.0 to 8.5, and more preferably 3.5 to 8.0, and the pH can be adjusted appropriately with a pH adjuster.

An administration route of the anti-fibrosis agent provided in the present invention includes oral administration, subcutaneous administration, intramuscular administration, intravenous administration, intraarterial administration, intrathecal administration, and intraperitoneal administration, depending on the dosage form.

Although the dose varies depending on a condition of a target patient (body weight, age, symptoms, physical condition, and the like), dosage form, and the like, the number of particles is usually 1 × 10⁵ to 1 × 10¹⁷ pieces/time, preferably 5 × 10⁵ to 5 × 10¹⁶ pieces/time, still more preferably 1 × 10⁶ to 1 × 10¹⁶ pieces/time, and particularly preferably 5 × 10⁶ to 5 × 10¹⁵ pieces/time but in a case of the administration to an adult. This dose may be used as a single dose and may be administered multiple times a day, and this dose may be divided into a plurality of times and administered.

### Examples

Hereinafter, the present invention will be specifically described based on Examples and Comparative Examples, but the present invention is not limited to these examples.

### Example 1. Acquisition of Extracellular Vesicle by PS-affinity method

### (1) Cell Culture

Poietics (trademark) human mesenchymal stem cells (Lonza.), which are bone marrow-derived mesenchymal stem cells, were cultured in MEMα (L-glutamine, phenol red contained, FUJIFILM Wako Pure Chemical Corporation) containing 15% FBS (Selborne Biological Services Pty. Co., Ltd.) used as a proliferated culture medium. Thereafter, the cultured bone marrow-derived mesenchymal stem cells were seeded in a 100 mm cell culture dish (Corning Incorporated) with a cell count of 3 × 10⁵, cultured in a cell culture incubator set under conditions of 5% CO₂ and 37°C for 72 hours, and proliferated to a cell count of 3 × 10⁶.

### (2) Production of Extracellular Vesicle

The culture medium was substituted with 20 mL D-MEM (FUJIFILM Wako Pure Chemical Corporation) containing 10% GIBCO, extracellular vesicle production culture medium: Fetal Bovine Serum, exosome-depleted, One Shot (trademark) format (Thermo Fisher Scientific Inc.), and the bone marrow-derived mesenchymal stem cells proliferated in (1) were cultured in a cell culture incubator set under conditions of 5% CO₂ and 37°C for 120 hours. Thereafter, the obtained culture supernatant was recovered into a 50 mL centrifuge tube and centrifuged at 2,000 × g for 20 minutes, and the supernatant was recovered.

### (3) Acquisition of Extracellular Vesicle by PS-affinity method

Extracellular vesicles were isolated from 1 mL of the culture supernatant recovered in (2) using MagCapture (trademark) Exosome Isolation Kit PS (FUJIFILM Wako Pure Chemical Corporation) according to a procedure described in the instruction manual attached to the kit to obtain extracellular vesicles in 1 mM EDTA-containing phosphate-buffered saline (PBS) to which an EV-Save (trademark) extracellular vesicle blocking reagent (FUJIFILM Wako Pure Chemical Corporation) was added. Thereafter, the buffer was exchanged with PBS to which EV-Save (trademark) extracellular vesicle blocking reagent was added, by using Vivaspin 500 (Sartorius AG, molecular weight cut-off: 100,000 (100K), membrane material: PES). Hereinafter, the obtained solution may be referred to as an "extracellular vesicle solution (PS-affinity method, bone marrow-derived MSC)".

### Comparative Example 1. Acquisition of Extracellular Vesicle by Ultracentrifugal method

The culture supernatant prepared according to the same method as in Example 1(1) and (2) was centrifuged at 110,000 × g for 70 minutes, by using an ultracentrifuge (Beckman: Optima (trademak) L-100XP). As a result, pellets were obtained, 1 mL of PBS was added to the obtained pellets, and the mixture was centrifuged again at 110,000 × g for 70 minutes. The finally obtained pellets were dissolved with PBS to which an EV-Save (trademark) extracellular vesicle blocking reagent was added (FUJIFILM Wako Pure Chemical Corporation). Hereinafter, the obtained solution may be referred to as an "extracellular vesicle solution (ultracentrifugal method, bone marrow-derived MSC)".

### Experimental Example 1. Measurement of The Number of Particles of Extracellular Vesicles by Nanotracking Analysis Method

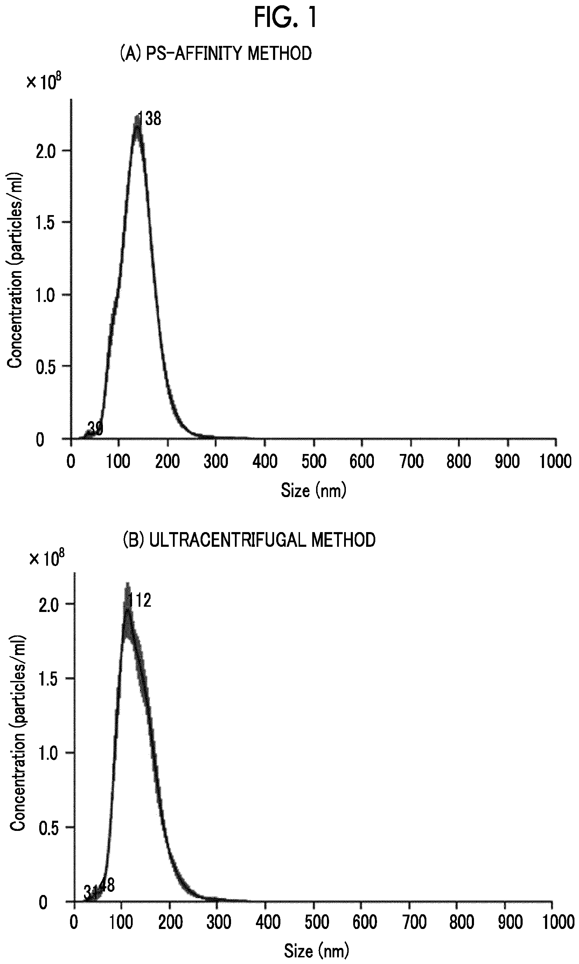

The number of particles of the "extracellular vesicle solution (PS-affinity method, bone marrow-derived MSC)" obtained in Example 1 per unit volume was measured by a nanoparticle tracking analysis method (nanotracking analysis method) using NanoSight (Malvern Panalytical Ltd) according to a procedure described in the manual of NanoSight, and an average particle size and an average number of particles per unit volume [particles/mL] were calculated. A graph of the obtained particle size distribution is illustrated in Fig. 1 together with the results of Experimental Example 2. In the graph of Fig. 1, the vertical axis indicates the number of particles, and the horizontal axis indicates the particle size. The average particle size of the extracellular vesicles acquired by the PS-affinity method was 138.1 ± 0.2 nm, and the average number of particles per unit volume was 1.76 × 10¹⁰ [particles/mL].

### Experimental Example 2. Measurement of The Number of Particles of Extracellular Vesicles by Nanotracking Analysis Method

An average particle size and an average number of particles per unit volume [particles/mL] were calculated according to the same method as in Experimental Example 1, except that the "extracellular vesicle solution (ultracentrifugal method, bone marrow-derived MSC)" obtained in Comparative Example 1 was used instead of the "extracellular vesicle solution (PS-affinity method, bone marrow-derived MSC)". A graph of the obtained particle size distribution is illustrated in Fig. 1 together with the results of Experimental Example 1. The average particle size of the extracellular vesicles acquired by the ultracentrifugal method was 138.1 ± 2.9 nm, and the average number of particles per unit volume was 1.68 × 10¹⁰ [particles/mL].

### Experimental Example 3. Analysis of Extracellular Vesicle Marker Protein by Western Blotting Method

Extracellular vesicle marker proteins CD9, CD63, and CD81 were analyzed by a Western blotting technique by using the "extracellular vesicle solution (PS-affinity method, bone marrow-derived MSC)" obtained in Example 1.

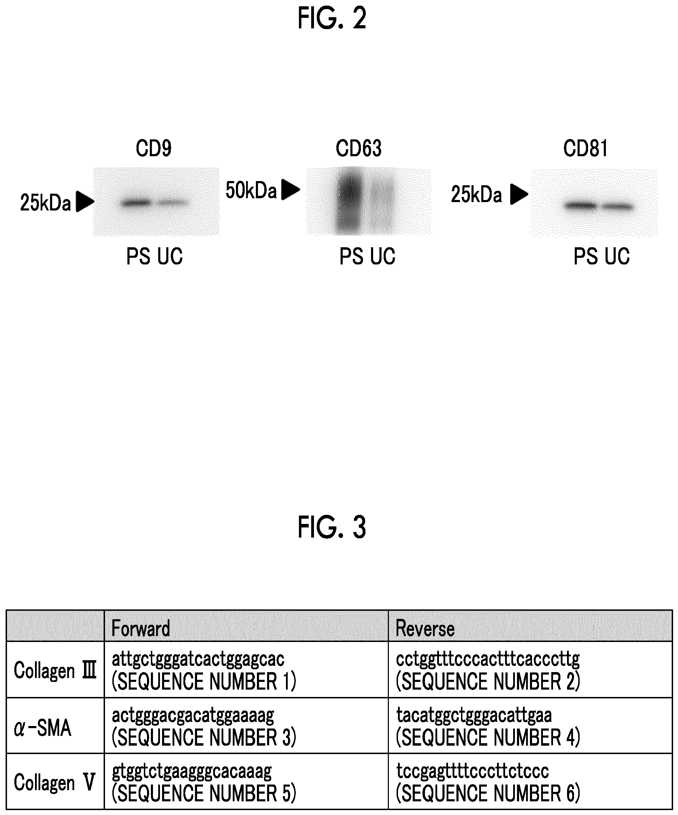

Based on the average number of particles per unit volume in the "extracellular vesicle solution (PS-affinity method, bone marrow-derived MSC)" calculated in Experimental Example 1, 1/4 amount of SDS-PAGE Sample Buffer 4-fold concentrated solution (without a reducing agent) was mixed with 3.0 × 10⁸ particles of the "extracellular vesicle solution (PS-affinity method, bone marrow-derived MSC)" (FUJIFILM Wako Pure Chemical Corporation), and the total amount was subjected to electrophoresis on a 10% to 20% acrylamide gel (FUJIFILM Wako Pure Chemical Corporation). Thereafter, the transcription was carried out to a PVDF membrane (Bio-Rad Laboratories, Inc.) by using a transfer buffer (FUJIFILM Wako Pure Chemical Corporation). The transferred membrane was immersed in a PBS-T solution containing 1% skim milk (0.1 (w/v)% Tween (trademark) 20-containing PBS solution) for 1 hour and was subjected to blocking treatment, and each of anti-CD9 antibodies (FUJIFILM Wako Pure Chemical Corporation), anti-CD63 antibodies (FUJIFILM Wako Pure Chemical Corporation), and anti-CD81 antibodies (FUJIFILM Wako Pure Chemical Corporation) was allowed to react with a primary antibody solution adjusted to 1.1 µg/mL with a PBS-T solution. Thereafter, the membrane on which the anti-CD9 antibodies and the anti-CD81 antibodies had reacted was allowed to react with a secondary antibody solution obtained by diluting HRP-flagged anti-rat IgG antibodies (Jackson ImmunoResearch Inc.) to 10,000 times with a PBS-T solution, and the membrane on which the anti-CD63 antibodies had reacted was allowed to react with a secondary antibody solution obtained by diluting HRP-flagged anti-mouse IgG antibodies (Agilent Technologies, Inc.) with a PBS-T solution to 10,000 times. Thereafter, Immunostar (trademark) Zeta (FUJIFILM Wako Pure Chemical Corporation) was used as a detection reagent to detect a signal by using Amersham Imager 600 (General Electric Company). The results of Western blotting are illustrated in Fig. 2 together with the results of Experimental Example 4. In the figure, PS on the horizontal axis represents the result in a case where the "extracellular vesicle solution (PS-affinity method, bone marrow-derived MSC)" is used (Experimental Example 3), and UC represents the result in a case where the "extracellular vesicle solution (ultracentrifugal method, bone marrow-derived MSC)" is used (Experimental Example 4). The arrows on the vertical axis indicate band positions of the detected extracellular vesicle marker proteins.

### Experimental Example 4. Analysis of Extracellular Vesicle Marker Protein by Western Blotting Method

Extracellular vesicle marker proteins CD9, CD63, and CD81 were analyzed by a Western blotting technique according to the same method as in Experimental Example 3, except that the "extracellular vesicle solution (ultracentrifugal method, bone marrow-derived MSCs)" obtained in Comparative Example 1 was used instead of the "extracellular vesicle solution (PS-affinity method, bone marrow-derived MSCs)". The result of Western blotting is illustrated in Fig. 2 together with the result of Experimental Example 3.

It was found from Fig. 2 that expression levels of CD9 and CD63 of the extracellular vesicles acquired by the PS-affinity method [extracellular vesicles in the "extracellular vesicle solution (PS-affinity method, bone marrow-derived MSC)", Experimental Example 3] were higher than those of the extracellular vesicles [extracellular vesicles in the "extracellular vesicle solution (ultracentrifugal method, bone marrow-derived MSC)", Experimental Example 4] obtained by the ultracentrifugal method.

### Examples 2 to 4. Evaluation of Anti-fibrosis Action of Extracellular Vesicle acquired by PS-affinity Method

The anti-fibrosis action of the extracellular vesicles in the "extracellular vesicle solution (PS-affinity method, bone marrow-derived MSC)" prepared in Example 1 was evaluated.

TIG3 cells (distributed from JCRB), which are human fetal lung-derived fibroblasts, were suspended in DMEM (FUJIFILM Wako Pure Chemical Corporation) containing 10% FBS (Biosera), and were seeded in 24-well plate (Corning Incorporated) with a cell count of 2.5 × 10⁴ per well and a medium volume of 500 µL. Twenty-four hours after cell seeding, the "extracellular vesicle solution (PS-affinity method, bone marrow-derived MSC)" obtained in Example 1 was added into each well in an amount to obtain a final concentration of 1 × 10⁹ particles with reference to the measurement result of Experimental Example 1, and cultured for 16 hours. Thereafter, an amount of TGFβ1 (R&D Systems, Inc.) to obtain a final concentration of 5 ng/mL was added to each well, stimulation was carried out, and furthermore culturing was carried out for 24 hours. Each RNA was extracted by using a PureLink (trademark) RNA Mini kit (Thermo Fisher Scientific Inc.) according to the procedure described in the instruction manual attached to the kit. From 200 ng of the extracted RNA, cDNA was synthesized by using RiverTra Ace (trademark) qPCR RT Master Mix with gDNA Remover (Toyobo Co., Ltd.) according to the procedure described in the instruction manual attached to the kit. A mRNA expression level of each of the collagen III (Example 2), collagen V (Example 3), α-SMA collagen (Example 4), which are fibrosis-related genes, and the internal standard GAPDH was measured by quantitative PCR, by KOD SYBR qPCR Mix (Toyobo Co., Ltd.) using the synthesized cDNA. Each of primers used for quantitative PCR is illustrated in Fig. 3 (SEQ ID NO. 1 to 6).

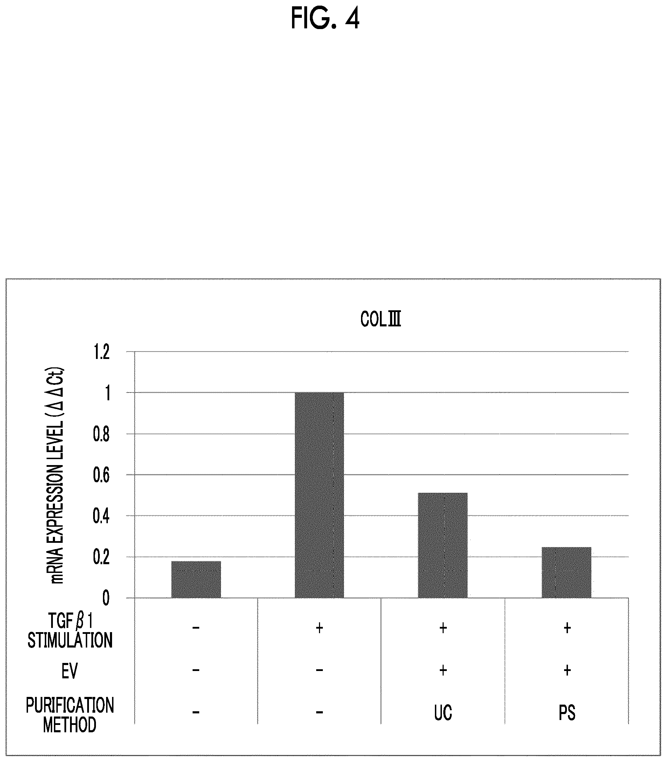

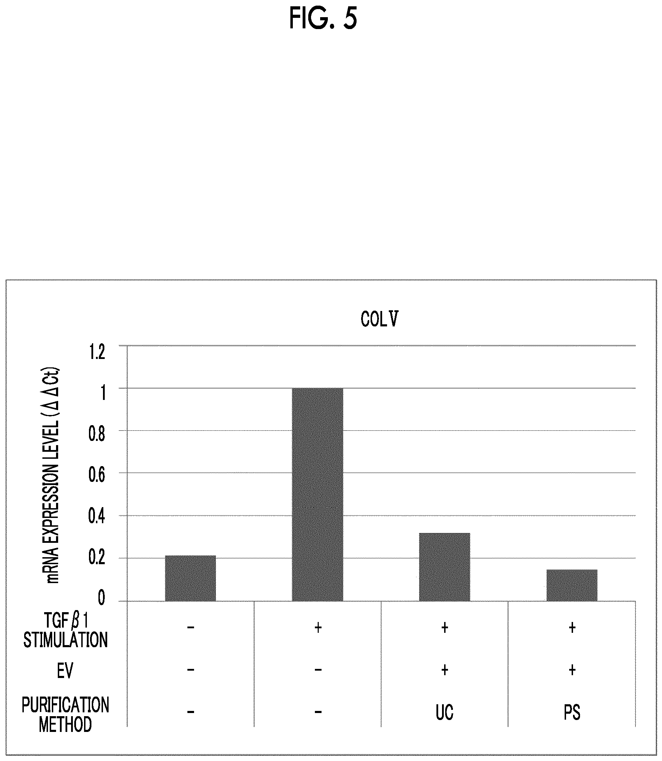

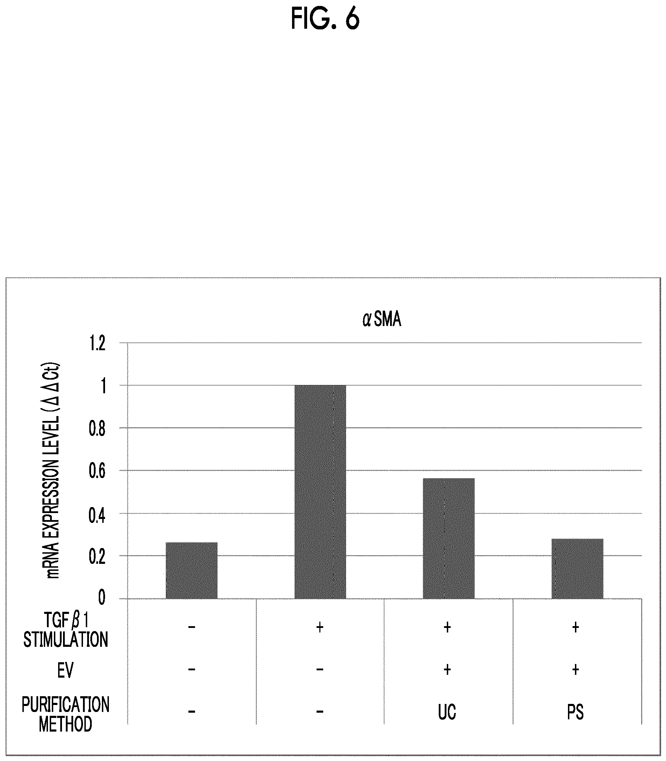

The results of quantitative PCR are illustrated in Figs. 4 to 6 together with the results of Comparative Examples 2 to 4. Fig. 4 illustrates the results of the mRNA expression level of collagen III, Fig. 5 illustrates the result of the mRNA expression level of collagen V, and Fig. 6 illustrates the result of the mRNA expression level of α-SMA. In the figures, in the horizontal axis, "TGFβ1 STIMULATION (-), EV (-), PURIFICATION METHOD (-)" illustrates the result in TIG3 to which stimulation was not applied, "TGFβ1 STIMULATION (+), EV (-), PURIFICATION METHOD (-)" illustrates the result in TIG3 (control) to which TGFβ1 stimulation was applied, "TGFβ1 STIMULATION (+), EV (+), PURIFICATION METHOD (PS)" illustrates the result in a case where the "extracellular vesicle solution (PS-affinity method, bone marrow-derived MSC) was used to TIG3 to which TGFβ1 stimulation was applied (Example 2 (Fig. 4), Example 3 (Fig. 5), and Example 4 (Fig. 6)), and "TGFβ1 STIMULATION (+), EV (+), PURIFICATION METHOD (UC)" illustrates the result in a case where the "extracellular vesicle solution (ultracentrifugal method, bone marrow-derived MSC)" was used to TIG3 to which TGFβ1 stimulation was applied (Comparative Example 2 (Fig. 4), Comparative Example 3 (Fig. 5), and Comparative Example 4 (Fig. 6)). As for the expression level of each gene, the numerical value obtained from qPCR performed with the primer for each gene was normalized to GAPDH, illustrated as the ΔΔct value relative to the expression of the target mRNA in the control, and illustrated on the vertical axis of each figure.

### Comparative Examples 2 to 4. Evaluation of Anti-fibrosis Action of Extracellular Vesicle acquired by Ultracentrifugal Method

A mRNA expression level of each of the collagen III (Comparative Example 2), collagen V (Comparative Example 3), α-SMA collagen (Comparative Example 4), which are fibrosis-related genes, and the internal standard GAPDH was measured by quantitative PCR according to the same method as in Examples 2 to 4, except that the "extracellular vesicle solution (ultracentrifugal method, bone marrow-derived MSC)" obtained in Comparative Example 1 was used instead of the "extracellular vesicle solution (PS-affinity method, bone marrow-derived MSCs)", to evaluate the anti-fibrosis action of the extracellular vesicles. The results of quantitative PCR are illustrated in Figs. 4 to 6 together with the results of Examples 2 to 4.

It was confirmed from Figs. 4 to 6 that the mRNA expression levels of all fibrosis-related genes were increased by TGFβ1 stimulation. It was found that such an increase in the mRNA expression level due to TGFβ was suppressed by using the extracellular vesicles acquired by the PS-affinity method (having the anti-fibrosis action). In addition, it was found that the extracellular vesicles acquired by the PS-affinity method (Examples 2 to 4) have a remarkably higher anti-fibrosis action (anti-fibrosis effect) than the extracellular vesicles acquired by the ultracentrifugal method (Comparative Examples 2 to 4).

### Examples 5 to 7. Evaluation of Anti-fibrosis Action of Extracellular Vesicle acquired by PS-affinity Method

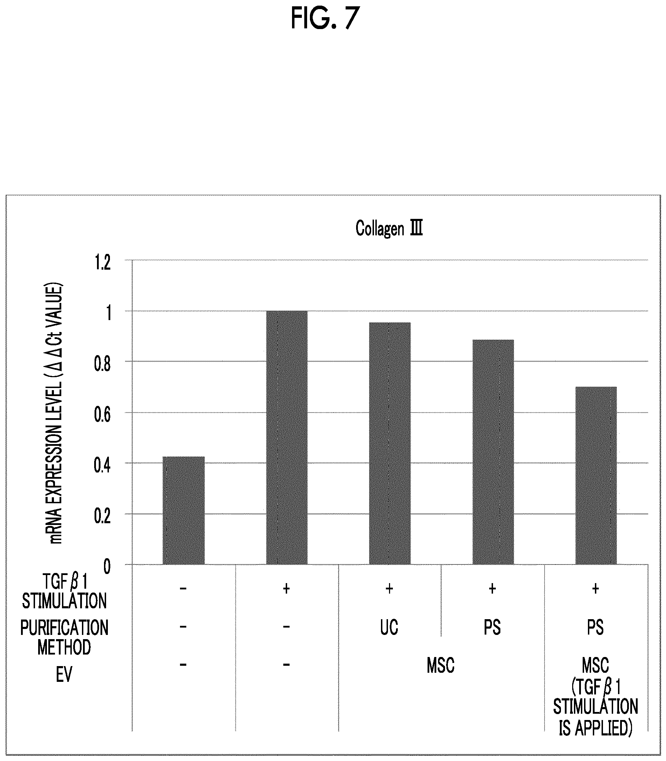

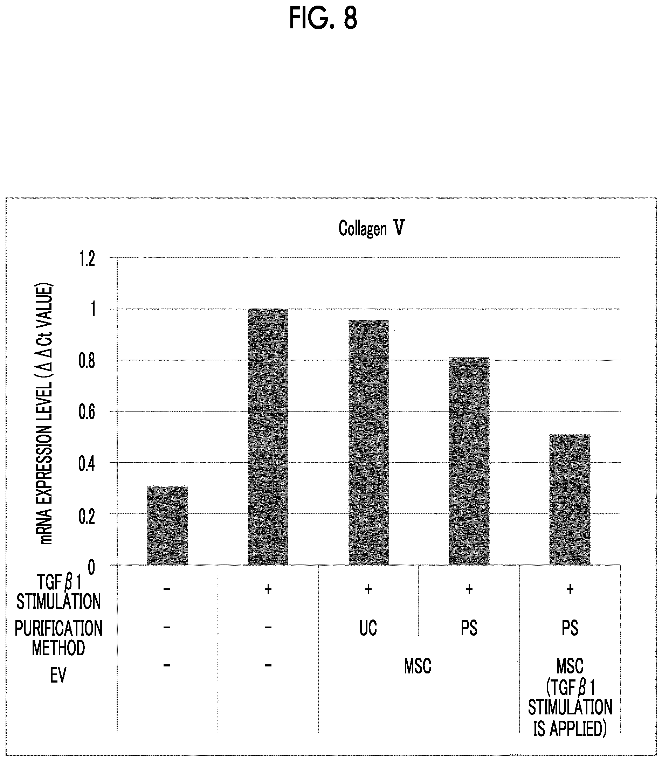

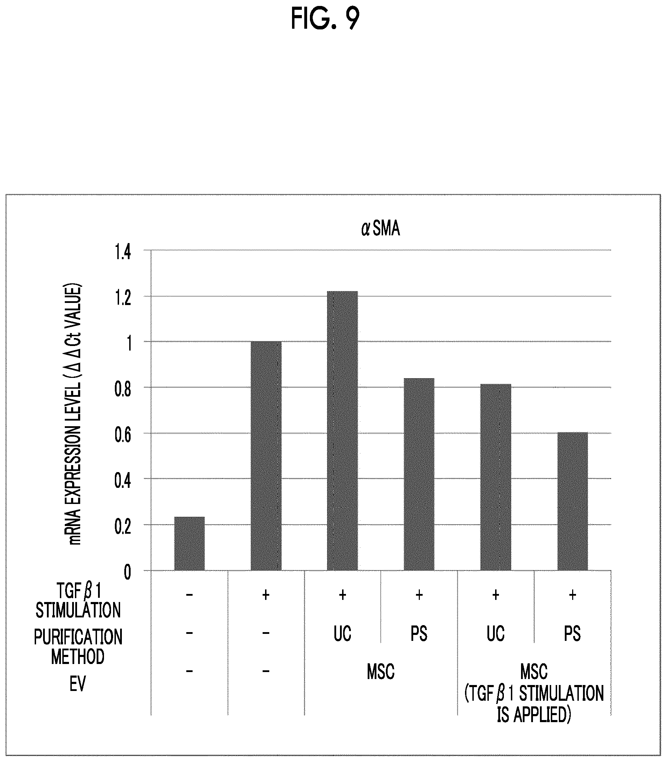

A mRNA expression level of each of the collagen III (Example 5), collagen V (Example 6), α-SMA collagen (Example 7), which are fibrosis-related genes, and the internal standard GAPDH was measured by quantitative PCR according to the same method as in Examples 2 to 4, except that the "extracellular vesicle solution (PS-affinity method, bone marrow-derived MSC)" obtained in Example 1 in an "amount to obtain a final concentration of 6 × 10⁸ particles/mL" was used instead of using the "extracellular vesicle solution (PS-affinity method, bone marrow-derived MSCs)" obtained in Example 1 in an "amount to obtain a final concentration of 1 × 10⁹ particles/mL", to evaluate the anti-fibrosis action of the extracellular vesicles acquired by the PS-affinity method. The results of quantitative PCR are illustrated in Figs. 7 to 9 together with the results of Examples 8 to 11, and Comparative Examples 5 to 7. Fig. 7 illustrates the results of the mRNA expression level of collagen III, Fig. 8 illustrates the result of the mRNA expression level of collagen V, and Fig. 9 illustrates the result of the mRNA expression level of α-SMA. The horizontal axis in the figures represents the same meaning as in Figs. 4 to 6.

### Comparative Examples 5 to 7. Evaluation of Anti-fibrosis Action of Extracellular Vesicle acquired by Ultracentrifugal Method

A mRNA expression level of each of the collagen III (Comparative Example 5), collagen V (Comparative Example 6), α-SMA collagen (Comparative Example 7), which are fibrosis-related genes, and the internal standard GAPDH was measured by quantitative PCR according to the same method as in Comparative Examples 2 to 4, except that the "extracellular vesicle solution (ultracentrifugal method, bone marrow-derived MSC)" obtained in Comparative Example 1 in an "amount to obtain a final concentration of 6 × 10⁸ particles/mL" was used instead of using the "extracellular vesicle solution (ultracentrifugal method, bone marrow-derived MSCs)" obtained in Comparative Example 1 in an "amount to obtain a final concentration of 1 × 10⁹ particles/mL", to evaluate the anti-fibrosis action of the extracellular vesicles acquired by the ultracentrifugal method. The results of quantitative PCR are illustrated in Figs. 7 to 9 together with the results of Examples 5 to 11.

### Examples 8 to 10. Evaluation of Anti-fibrosis Action of Extracellular Vesicle derived from TGFβ1-stimulated Mesenchymal Stem Cell acquired by PS-affinity Method

Bone marrow-derived mesenchymal stem cells were proliferated by the same method as in Example 1 (1) Cell Culture, and TGFβ1 (R&D Systems, Inc.) was added at a final concentration of 20 ng/mL, stimulation was carried out, and furthermore culturing was carried out for 24 hours. Next, extracellular vesicles were isolated according to the same method as in Example 1 (2) Production of Extracellular Vesicle and Example 1 (3) Acquisition of Extracellular Vesicle by PS-affinity method, and the buffer was exchanged with PBS to which a EV-Save (trademark) extracellular vesicle blocking reagent was added. Hereinafter, the obtained solution may be referred to as an "extracellular vesicle solution (PS-affinity method, bone marrow-derived MSC, TGFβ1 stimulation)". The average particle size and the average number of particles per unit volume [particles/mL] of the obtained "extracellular vesicle solution (PS-affinity method, bone marrow-derived MSC, TGFβ1 stimulation)" were calculated by the same method as in Experimental Example 1. In addition, a mRNA expression level of each of the collagen III (Example 8), collagen V (Example 9), α-SMA collagen (Example 10), which are fibrosis-related genes, and the internal standard GAPDH was measured by quantitative PCR according to the same method as in Examples 2 to 4, except that the "extracellular vesicle solution (PS-affinity method, bone marrow-derived MSC, TGFβ1 stimulation)" was used instead of the "extracellular vesicle solution (PS-affinity method, bone marrow-derived MSCs)", to evaluate the anti-fibrosis action of the extracellular vesicles acquired by the PS-affinity method. The results of quantitative PCR are illustrated in Figs. 7 to 9 together with the results of Examples 5 to 7 and 11, and Comparative Examples 5 to 7.

### Example 11. Evaluation of Anti-fibrosis Action of Extracellular Vesicle derived from TGFβ1-stimulated Mesenchymal Stem Cell acquired by Ultracentrifugal Method

Bone marrow-derived mesenchymal stem cells were proliferated by the same method as in Example 1 (1) Cell Culture, and TGFβ1 (R&D Systems, Inc.) was added at a final concentration of 20 ng/mL, stimulation was carried out, and furthermore culturing was carried out for 24 hours. Next, the culture supernatant prepared according to the same method as in Example 1(2) Production of Extracellular Vesicle was centrifuged at 110,000 × g for 70 minutes, by using an ultracentrifuge (Beckman: Optima (trademak) L-100XP). As a result, pellets were obtained, 1 mL of PBS was added to the obtained pellets, and the mixture was centrifuged again at 110,000 × g for 70 minutes. The finally obtained pellets were dissolved with PBS to which an EV-Save (trademark) extracellular vesicle blocking reagent was added (FUJIFILM Wako Pure Chemical Corporation). Hereinafter, the obtained solution may be referred to as an "extracellular vesicle solution (ultracentrifugal method, bone marrow-derived MSC, TGFβ1 stimulation)". The average particle size and the average number of particles per unit volume [particles/mL] of the obtained "extracellular vesicle solution (PS-affinity method, bone marrow-derived MSC, TGFβ1 stimulation)" were calculated by the same method as in Experimental Example 1. In addition, a mRNA expression level of α-SMA collagen (Example 11) that is a fibrosis-related gene, and the internal standard GAPDH was measured by quantitative PCR according to the same method as in Example 4, except that the "extracellular vesicle solution (ultracentrifugal method, bone marrow-derived MSC, TGFβ1 stimulation)" was used instead of the "extracellular vesicle solution (PS-affinity method, bone marrow-derived MSCs)", to evaluate the anti-fibrosis action of the extracellular vesicles acquired by the PS-affinity method. The results of quantitative PCR are illustrated in Figs. 7 to 9 together with the results of Examples 5 to 10, and Comparative Examples 5 to 7.

It was found from Figs. 7 to 9 that an increase in the mRNA expression levels of the fibrosis-related genes due to TGFβ was suppressed by using the extracellular vesicles acquired by the PS-affinity method (the extracellular vesicles acquired by the PS-affinity method have the anti-fibrosis action). In addition, it was found that the extracellular vesicles acquired by the PS-affinity method (Examples 5 to 7) have a remarkably higher anti-fibrosis action than the extracellular vesicles acquired by the ultracentrifugal method (Comparative Examples 5 to 7). It was found from Figs. 7 to 9 that the extracellular vesicles (Examples 8 to 13) acquired from the mesenchymal stem cells stimulated with TGFβ had a higher anti-fibrosis action than the extracellular vesicles acquired from the mesenchymal stem cells (Examples 5 to 7, Comparative Examples 5 to 7) to which stimulation was not applied. Furthermore, the extracellular vesicles (Example 10) acquired from the mesenchymal stem cells stimulated with TGFβ by the PS-affinity method had a particularly remarkable anti-fibrosis action than the extracellular vesicles (Example 11) acquired by another means, such as extracellular vesicles acquired from the mesenchymal stem cells stimulated with TGFβ by the ultracentrifugal method.

### Examples 12 and 13: Acquisition of Extracellular Vesicle by PS-affinity Method

Extracellular vesicles were acquired by the same method as in Example 1 except that "human adipose-derived stem cells (Lonza.) that are adipose-derived mesenchymal stem cells" (Example 12) or "human mesenchymal stem cells from umbilical cord matrix (PromoCell GmbH), which are umbilical cord-derived mesenchymal stem cells" (Example 13) was used instead of "Poietics (trademark) human mesenchymal stem cells that are bone marrow-derived mesenchymal stem cells" (Lonza.), and an "extracellular vesicle solution (PS-affinity method, adipose-derived MSC)" (Example 12) and an "extracellular vesicle solution (PS-affinity method, umbilical cord-derived MSC)" (Example 13) were individually obtained.

### Comparative example 8. Acquisition of extracellular vesicle by Ultracentrifugal Method

The culture supernatant prepared according to the same method as in Example 1(1) and (2) was centrifuged at 110,000 × g for 70 minutes, by using an ultracentrifuge (Beckman: Optima (trademark) L-100XP), except that "human adipose-derived stem cells (Lonza.) that are adipose-derived mesenchymal stem cells" was used instead of "Poietics (trademark) human mesenchymal stem cells that are bone marrow-derived mesenchymal stem cells (Lonza.). As a result, pellets were obtained, 1 mL of PBS was added to the obtained pellets, and the mixture was centrifuged again at 110,000 × g for 70 minutes. The finally obtained pellets were dissolved with PBS to which an EV-Save (trademark) extracellular vesicle blocking reagent was added (FUJIFILM Wako Pure Chemical Corporation), to obtain the "extracellular vesicle solution (ultracentrifugal method, adipose-derived MSC)"

### Examples 14 to 19. Evaluation of Anti-fibrosis Action of Extracellular Vesicle acquired by PS-affinity Method

The anti-fibrosis action of the extracellular vesicles in the "extracellular vesicle solution (PS-affinity method)" prepared in Examples 12 and 13 was evaluated.

A mRNA expression level of each of the collagen III (Example 14), collagen V (Example 16), α-SMA collagen (Example 18), which are fibrosis-related genes, and the internal standard GAPDH was measured by quantitative PCR according to the same method as in Examples 5 to 7, except that the "extracellular vesicle solution (PS-affinity method, adipose-derived MSC)" obtained in Example 12 was used instead of the "extracellular vesicle solution (PS-affinity method, bone marrow-derived MSCs)". Similarly, a mRNA expression level of each of the collagen III (Example 15), collagen V (Example 17), α-SMA collagen (Example 19), and the internal standard GAPDH was measured by quantitative PCR according to the same method as in Examples 5 to 7, except that the "extracellular vesicle solution (PS-affinity method, umbilical cord-derived MSC)" obtained in Example 13 was used instead of the "extracellular vesicle solution (PS-affinity method, bone marrow-derived MSCs)". Each of primers used for quantitative PCR is illustrated in Fig. 3 (SEQ ID NO. 1 to 6).

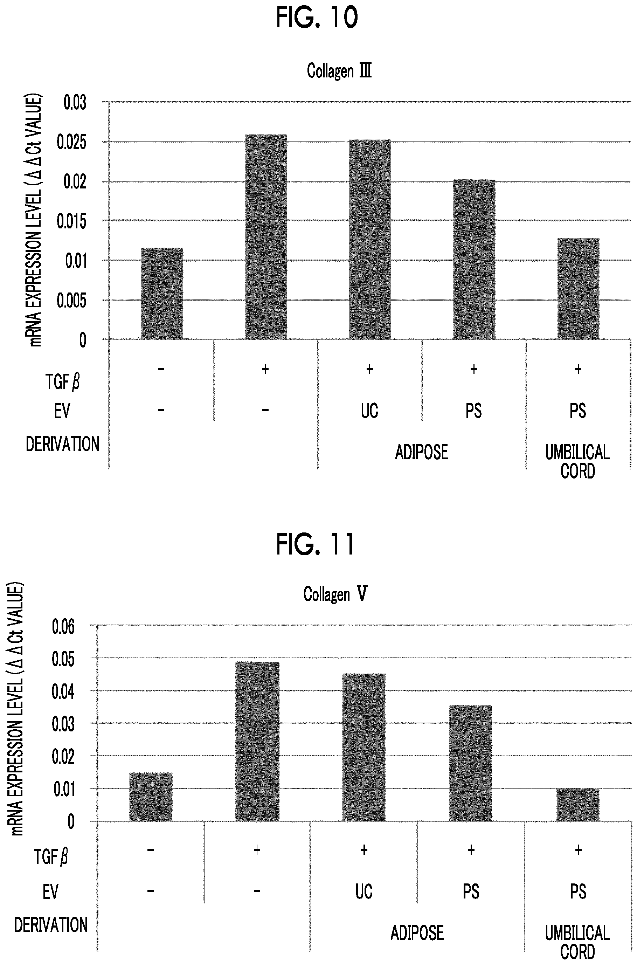

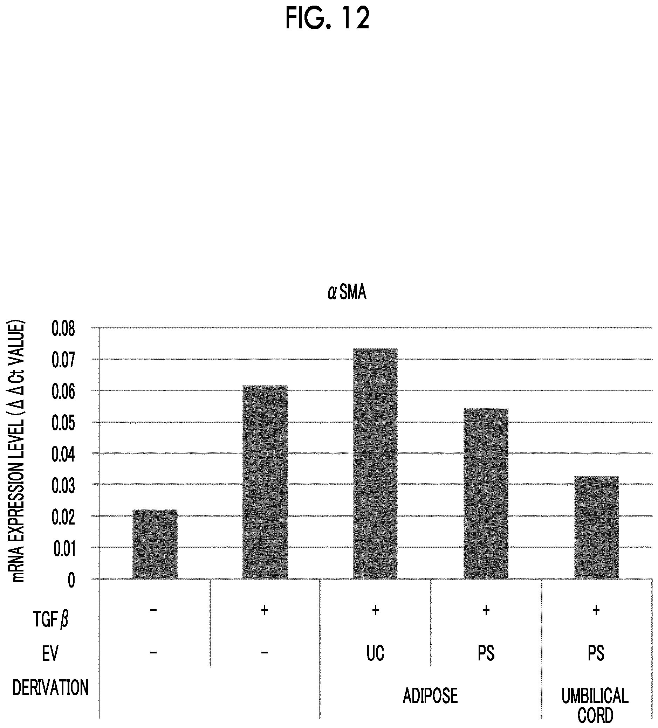

The results of quantitative PCR are illustrated in Figs. 10 to 12 together with the results of Comparative Examples 9 to 11. Fig. 10 illustrates the results of the mRNA expression level of collagen III, Fig. 11 illustrates the result of the mRNA expression level of collagen V, and Fig. 12 illustrates the result of the mRNA expression level of α-SMA. In the figures, in the horizontal axis, "TGFβ (-), EV (-), DERIVATION (-)" illustrates the result in TIG3 to which stimulation was not applied, "TGFβ STIMULATION (+), EV (-), DERIVATION (-)" illustrates the result in TIG3 (control) to which TGFβ1 stimulation was applied, "TGFβ STIMULATION (+), EV (PS), DERIVATION (ADIPOSE)" illustrates the result in a case where the "extracellular vesicle solution (PS-affinity method, adipose-derived MSC) was used to TIG3 to which TGFβ1 stimulation was applied (Example 14 (Fig. 10), Example 16 (Fig. 11), and Example 18 (Fig. 12)), and "TGFβ STIMULATION (+), EV (UC), DERIVATION (ADIPOSE)" illustrates the result in a case where the "extracellular vesicle solution (ultracentrifugal method, adipose-derived MSC)" was used to TIG3 to which TGFβ1 stimulation was applied [Comparative Example 9 (Fig. 10), Comparative Example 10 (Fig. 11), and Comparative Example 11 (Fig. 12)], and "TGFβ STIMULATION (+), EV (PS), DERIVATION (UMBILICAL CORD)" illustrates the result in a case where the "extracellular vesicle solution (PS-affinity method, umbilical cord-derived MSC)" was used to TIG3 to which TGFβ1 stimulation was applied [Example 15 (Fig. 10), Example 17 (Fig. 11), and Example 19 (Fig. 12)]. As for the expression level of each gene, the numerical value obtained from qPCR performed with the primer for each gene was normalized to GAPDH, illustrated as the ΔΔct value relative to the expression of the target mRNA in the control, and illustrated on the vertical axis of each figure.

### Comparative Examples 9 to 11. Evaluation of Anti-fibrosis Action of Extracellular Vesicle acquired by Ultracentrifugal Method

A mRNA expression level of each of the collagen III (Comparative Example 9), collagen V (Comparative Example 10), α-SMA collagen (Comparative Example 11), which are fibrosis-related genes, and the internal standard GAPDH was measured by quantitative PCR according to the same method as in Examples 14 to 19, except that the "extracellular vesicle solution (ultracentrifugal method, adipose-derived MSCs)" was used instead of the "extracellular vesicle solution (PS-affinity method, bone marrow-derived MSCs)". Each of primers used for quantitative PCR is illustrated in Fig. 3 (SEQ ID NO. 1 to 6). The results of quantitative PCR are illustrated in Figs. 10 to 12 together with the results of Examples 14 to 19.

It was found from Figs. 10 to 12 that an increase in the mRNA expression level of the fibrosis-related genes due to TGFβ is suppressed even in the case where the "extracellular vesicle solution (PS-affinity method, adipose-derived MSC)" was used (Examples 14, 16, and 18) and the case where the "extracellular vesicle solution (PS-affinity method, umbilical cord-derived MSC)" were used (Examples 15, 17, and 19), that is, the extracellular vesicles acquired by the PS-affinity method have the anti-fibrosis action. Based on these results, it was found that the extracellular vesicles (Examples 14 to 19) acquired by the PS-affinity method have a remarkably higher anti-fibrosis action than the extracellular vesicles (Comparative Examples 9 to 11) obtained by the ultracentrifugal method, regardless of the type of mesenchymal stem cells.

The present invention is to provide the anti-fibrosis agent containing an extracellular vesicle that has an anti-fibrosis action and that is acquired from a mesenchymal stem cell-derived extracellular vesicle, as an active ingredient. Since the anti-fibrosis agent provided in the present invention has a factor related to fibrosis of a living tissue or an organ, for example, an action of regulating the production of a fibrosis-related gene, it is possible to suppress the progress of fibrosis of the living tissue or the organ. Thus, the present invention has great industrial availability in that an effective therapeutic medication for diseases that cause fibrosis is provided.

### [Sequence list]

2157_ST250002.txt

## Claims

1. An anti-fibrosis agent comprising:
a mesenchymal stem cell-derived extracellular vesicle, as an active ingredient,
wherein an extracellular vesicle is obtained by a method of using a substance having an affinity for phosphatidylserine.

2. The anti-fibrosis agent according to claim 1, wherein the mesenchymal stem cell is a cell derived from an iPS cell or a cell derived from one or more tissues selected from the group consisting of umbilical cord, umbilical cord blood, bone marrow, adipose, muscle, nerve, skin, amniotic membrane, and placenta.

3. The anti-fibrosis agent according to claim 1, wherein the extracellular vesicle is obtained by the method of using a substance having an affinity for phosphatidylserine.

4. The anti-fibrosis agent according to claim 3, wherein the substance having an affinity for phosphatidylserine is a Tim protein.

5. The anti-fibrosis agent according to claim 4, wherein the Tim protein is selected from Tim4 protein, Tim3 protein, and Tim1 protein.

6. A method of producing an extracellular vesicle having an anti-fibrosis action, the method comprising a step of obtaining an extracellular vesicle from a mesenchymal stem cell-derived extracellular vesicle by a method of using a substance having an affinity for phosphatidylserine.

7. The method of producing an extracellular vesicle according to claim 6, wherein the substance having an affinity for phosphatidylserine is a Tim protein.

8. The method of producing an extracellular vesicle according to claim 7, wherein the Tim protein is selected from Tim4 protein, Tim3 protein, and Tim1 protein.
